(19) Europäisches Patentamt
European Patent Office
Office européen des brevets

(11) Numéro de publication : 0 304 649 B1

(12) # FASCICULE DE BREVET EUROPEEN

(45) Date de publication du fascicule du brevet : **20.03.91 Bulletin 91/12**

(51) Int. Cl.$^5$ : **C07D 239/50,** C07D 279/12, A61K 7/06, A61K 31/505, A61K 9/22, A61K 47/00

(21) Numéro de dépôt : **88112024.0**

(22) Date de dépôt : **26.07.88**

(54) **Sels du piperidino-6, diamino-2,4 pyrimidine oxyde-3 et de dérivés de l'acide thiamorpholinone-3 carboxylique-5, leur utilisation en cosmétique et pharmacie.**

Le dossier contient des informations techniques présentées postérieurement au dépôt de la demande et ne figurant pas dans le présent fascicule.

(30) Priorité : **31.07.87 LU 86958**

(43) Date de publication de la demande : **01.03.89 Bulletin 89/09**

(45) Mention de la délivrance du brevet : **20.03.91 Bulletin 91/12**

(84) Etats contractants désignés : **AT BE CH DE ES FR GB GR IT LI NL SE**

(56) Documents cités :
**EP-A- 0 211 268**
**GB-A- 2 032 434**
**GB-A- 2 118 553**
**Die pharmazeutische Industrie, (1985), 47, page 506, 2. colonne, 2 Abs.**

(73) Titulaire : **L'OREAL**
**14, Rue Royale**
**F-75008 Paris (FR)**

(72) Inventeur : **Lang, Gérard**
**44, Avenue Lacour**
**F-95210 Saint-Gratien (FR)**
Inventeur : **Maignan, Jean**
**8, Rue Halévy**
**F-93290 Tremblay les Gonesse (FR)**
Inventeur : **Restle, Serge**
**140 Rue Anatole France**
**F-93601 Aulnay sous Bois (FR)**

(74) Mandataire : **Casalonga, Axel**
**BUREAU D.A. CASALONGA - JOSSE**
**Morassistrasse 8**
**W-8000 München 5 (DE)**

EP 0 304 649 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Les dérivés de diamino-2,4 pyrimidine sont bien connus dans l'état de la technique et en particulier le pipéridino-6 diamino-2,4 pyrimidine oxyde-3 connu sous la dénomination de "Minoxidil". Ce produit est notamment utilisé en thérapeutique humaine pour ses propriétés antihypertensives ainsi que dans des applications topiques pour des traitements de la calvitie et de la pelade.

Le "Minoxidil" présente cependant l'inconvénient d'être pratiquement insoluble dans l'eau, ce qui limite son utilisation dans la mesure où les formulations mettent en oeuvre des proportions d'eau relativement faibles qui contiennent, par contre, de fortes concentrations en alcool ou polyol qui sont généralement moins bien tolérées.

On connaît, par ailleurs, déjà certains sels de "Minoxidil" et notamment le sulfate, le sel de N-acylthiazolidine-4 carboxylique, de L-5-oxoproline qui sont mentionnés respectivement dans les brevets W0 04231 (UPJOHN), FR 2852306 (SERONO) et DE 3617124 (SERONO).

La demanderesse vient de découvrir, ce qui fait l'objet de l'invention, que les sels de dérivés de l'acide thiamorpholinone-3 carboxylique-5 et de "Minoxidil" étaient particulièrement intéressants.

Les acides thiamorpholinone carboxyliques sont connus en eux-mêmes comme il est décrit dans le brevet français 2.525.220 de la demanderesse.

Ces acides sont essentiellement connus pour leurs propriétés hydratantes et émollientes.

Les sels de dérivés d'acide thiamorpholinone-3 carboxylique-5 et de pipéridino-6 diamino-2,4 pyrimidine oxyde-3, conformes à l'invention, se sont révélés être particulièrement solubles dans des milieux aqueux, ce qui permet notamment de remédier aux inconvénients des formulations antérieures, notamment en ce qui concerne leur tolérance par l'organisme.

Les sels présentent, par ailleurs, simultanément une activité sur la repousse des cheveux ainsi que sur la séborrhée. Ces propriétés font qu'ils sont particulièrement bien appropriés pour le traitement du cuir chevelu, notamment le traitement de la chute des cheveux, mais également la pelade, les dermatites desquamantes, l'alopécie, etc.

L'invention a donc pour objet ce nouveaux sels de pipéridino-6 diamino-2,4 pyrimidine oxyde-3 constitués par les sels de dérivés de l'acide thiamorpholinone-3 carboxylique-5.

Un autre objet de l'invention est constitué par les compositions cosmétiques ou pharmaceutiques à base de ces sels.

L'invention a également pour objet un procédé de traitement du cuir chevelu mettant en oeuvre de tels sels.

D'autres objets de l'invention apparaîtront à la lecture de la description et des exemples qui suivent.

Les sels conformes à l'invention sont essentiellement caractérisés par le fait qu'ils répondent à la formule:

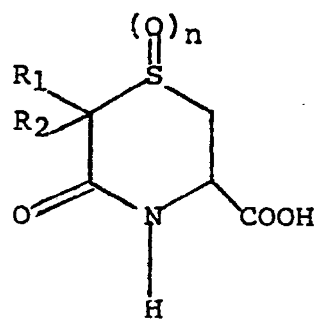

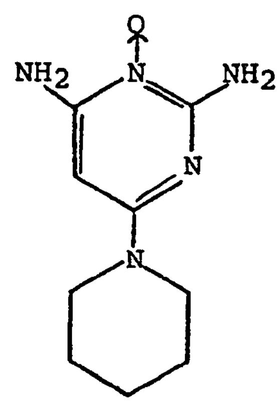

I(A)

ou à la forme ionique correspondante :

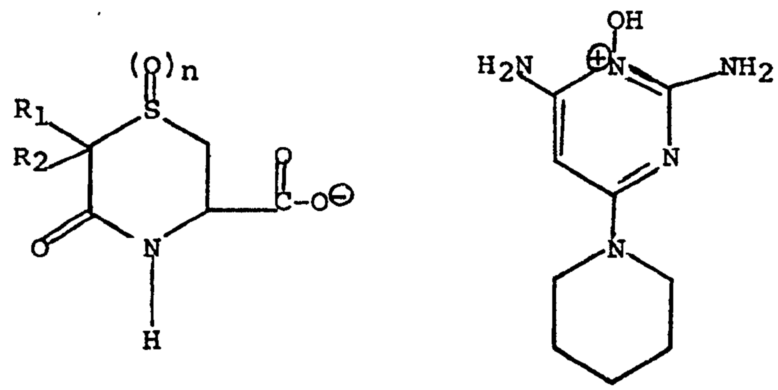

I(B)

dans laquelle :

n est égal à 0, 1 ou 2, et

$R_1$ et $R_2$ identiques ou différents, désignent un atome d'hydrogène ou un radical alkyle inférieur, linéaire ou ramifié, ayant de 1 à 6 atomes de carbone.

Par radical alkyle inférieur, on entend de préférence les radicaux méthyle, éthyle, propyle, isopropyle, butyle, pentyle et hexyle.

Les composés plus particulièrement préférés selon l'invention sont le thiamorpholinone-3 carboxylate-5 de pipéridino-6 diamino-2,4 hydroxy-3 pyrimidinium, le méthyl-2 thiamorpholinone-3 carboxylate-5 de pipéridino-6 diamino-2,4 hydroxy-3 pyrimidinium, le diméthyl-2,2 thiamorpholinone-3 carboxylate-5 de pipéridino-6 diamino-2,4 hydroxy-3 pyrimidinium, l'oxo-1 thiamorpholinone-3 carboxylate-5 de pipéridino-6 diamino-2,4 hydroxy-3 pyrimidinium, le dioxo-1,1 thiamorpholinone-3 carboxylate-5 de pipéridino-6 diamino-2,4 hydroxy-3 pyrimidinium, l'oxo-1 méthyl-2 thiamorpholinone-3 carboxylate-5 de pipéridino-6 diamino-2,4 hydroxy-3 pyrimidinium, l'oxo-1 diméthyl-2,2 thiamorpholinone-3 carboxylate-5 de pipéridino-6 diamino-2,4 hydroxy-3 pyrimidinium, l'hexyl-2 thiamorpholinone-3 carboxylate-5 de pipéridino-6 diamino-2,4 hydroxy-3 pyrimidinium.

Les compositions destinées à une application topique conformes à l'invention sont essentiellement caractérisées par le fait qu'elles contiennent 0,1 à 10% en poids d'au moins un composé de formule (I) et de préférence 0,5 à 5% en poids par rapport au poids total de la composition dans un milieu aqueux approprié pour une application topique.

Les compositions peuvent, en particulier, se présenter sous forme de lotions, de crèmes, de gels et éventuellement être conditionnées dans des dispositifs aérosols.

Ces compositions peuvent contenir, en plus de l'agent actif de formule (I), différents ingrédients classiquement utilisés dans les formulations cosmétiques ou pharmaceutiques destinées à une application topique.

Elles peuvent en particulier contenir du glycérol, de l'urée, de l'acide lactique, mais également des agents épaississants, des agents tensio-actifs, des solvants acceptables sur le plan cosmétique ou pharmaceutique, tels que des alcools inférieurs comme l'éthanol ou l'isopropanol, de l'éthylèneglycol, les éthers monométhylique, monoéthylique ou monobutylique de l'éthylèneglycol, le propylèneglycol, les monométhyléthers du propylèneglycol et du dipropylène glycol.

Ces compositions peuvent également contenir d'autres agents actifs, notamment des agents actifs sur le plan cosmétique tels que la thiamorpholinone, la S-benzylcystéamine et leurs dérivés, la tioxolone.

La demanderesse a découvert que grâce aux composés conformes à l'invention, il était possible de préparer une composition aqueuse présentant une meilleure tolérance pour l'application topique.

Grâce aux sels conformes à l'invention, il est également possible de diminuer la concentration en matière active du fait de leur plus grande biodisponibilité.

Ces compositions présentent, par ailleurs, l'avantage d'avoir des propriétés cosmétiques meilleures, les compositions de traitement ne poissant pas les cheveux et pouvant se rincer plus facilement.

Elles sont enfin beaucoup moins irritantes au niveau cutané et oculaire et moins toxiques.

Un autre objet de l'invention est constitué par le procédé de traitement cosmétique du cuir chevelu pour induire et stimuler la croissance des cheveux ou diminuer leur chute et combattre la séborrhée.

Ce procédé consiste essentiellement à appliquer les compositions telles que définies ci-dessus sur des zones alopéciques du cuir chevelu et les cheveux d'un individu.

L'application peut se faire par exemple après lavage du cuir chevelu et des cheveux à l'aide d'un shampooing ou peu de temps après un shampooing.

Les composés conformes à l'invention sont préparés,

– soit en ajoutant une solution alcoolique de l'acide thiamorpholinone carboxylique dans une solution

alcoolique préparée au préalable contenant un équivalent de pipéridino-6 diamino-2,4 pyrimidine oxyde-3, le mélange est alors concentré jusqu'au moment où le sel commence à cristalliser et l'ensemble est alors glacé ;

– soit en ajoutant un équivalent d'acide thiamorpholinone carboxylique sous forme solide à une suspension de pipéridino-6 diamino-2,4 pyrimidine oxyde-3 dans l'eau. On opère dans ce dernier cas dans un milieu très concentré et le sel est essoré et séché.

Les exemples suivants sont destinés à illustrer l'invention sans pour autant présenter un caractère limitatif.

## EXEMPLE 1

Synthèse du sel de l'acide thiamorpholinone-3 carboxylique-5 et du pipéridino-6 diamino-2,4 pyrimidine oxyde-3.

A une solution de 5 g de pipéridino-6 diamino-2,4 pyrimidine oxyde-3 dans 300 $cm^3$ de méthanol chaud, on ajoute une solution de 3,80 g d'acide thiamorpholinone-3 carboxylique-5 dans 70 $cm^3$ de méthanol bouillant.

On récupère 6,7 g de produit dont le spectre $^1H$ RMN est conforme à la structure attendue.

Analyse élémentaire : $C_{14}H_{22}N_6O_4S$

| | | C% | H% | N% | O% | S% |
|---|---|---|---|---|---|---|
| Théorie | : | 45,39 | 5,99 | 22,69 | 17,28 | 8,65 |
| Trouvé | : | 45,44 | 5,94 | 22,62 | 17,45 | 8,81 |

## EXEMPLE 2

Synthèse du sel de l'acide thiamorpholinone-3 carboxylique-5 et du pipéridino-6 diamino-2,4 pyrimidine oxyde-3.

A une suspension de 1 g de pipéridino-6 diamino-2,4 pyrimidine oxyde-3 dans 10 $cm^3$ d'eau, on ajoute 0,770 g d'acide thiamorpholinone-3 carboxylique-5, on maintient l'agitation pendant 1 heure puis on abandonne pendant une nuit.

On filtre le produit et on sèche sous vide. Après recristallisation dans le méthanol, on obtient un produit blanc qui fond à 205°C et dont le spectre $^1H$ RMN est conforme à la structure du sel obtenu dans l'exemple 1.

## EXEMPLE 3

Synthèse du sel de l'acide méthyl-2 thiamorpholinone-3 carboxylique-5 et du pipéridino-6 diamino-2,4 pyrimidine oxyde-3.

A une solution de 5 g de pipéridino-6 diamino-2,4 pyrimidine oxyde-3 dans 300 $cm^3$ de méthanol chaud, on ajoute une solution de 4,18 g d'acide méthyl-2 thiamorpholinone-3 carboxylique-5 dans le minimum de méthanol bouillant.

Le méthanol est évaporé sous pression réduite. Le sel attendu cristallise dans l'éther.

Par recristallisation dans un mélange isopropanol-éther diisopropylique, on obtient 6,65 g de sel de l'acide méthyl-2 thiamorpholinone-3 carboxylique-5 et du pipéridino-6 diamino-2,4 pyrimidine oxyde-3 dont le point de fusion est de 129-130°C.

Analyse élémentaire : $C_{15}H_{24}N_6O_4S$

| | | C% | H% | N% | O% | S% |
|---|---|---|---|---|---|---|
| Théorie | : | 46,86 | 6,29 | 21,86 | 16,65 | 8,34 |
| Trouvé | : | 46,63 | 6,31 | 21,69 | 16,87 | 8,20 |

EXEMPLE 4

Synthèse du sel de l'acide diméthyl-2,2 thiamorpholinone-3 carboxylique-5 et du pipéridino-6 diamino-2,4 pyrimidine oxyde-3.

A une solution de 1,10 g de pipéridino-6 diamino-2,4 pyrimidine oxyde-3 dans environ 100 cm³ de méthanol chaud, on ajoute une solution de 1 g de l'acide diméthyl-2,2 thiamorpholinone-3 carboxylique-5 dans le minimum de méthanol bouillant.

Le méthanol est évaporé sous pression réduite. Le sel attendu cristallise dans l'éther.

Par recristallisation dans un mélange isopropanol-éther diisopropylique, on obtient 1,16 g de sel de l'acide diméthyl-2,2 thiamorpholinone-3 carboxylique-5 et du pipéridino-6 diamino-2,4 pyrimidine oxyde-3 dont le point de fusion est de 160-161°C.

Analyse élémentaire : $C_{16}H_{26}N_6O_4S$

| | C% | H% | N% | O% | S% |
|---|---|---|---|---|---|
| Théorie : | 48,22 | 6,58 | 21,09 | 16,06 | 8,05 |
| Trouvé : | 48,29 | 6,68 | 20,96 | 16,03 | 8,10 |

EXEMPLE 5

Synthèse du sel de l'acide thiamorpholinone-3 oxyde-1 carboxylique-5 et du piperidino-6 diamino-2,4 pyrimidine oxyde-3

A une solution de 5 g de pipéridino-6 diamino-2,4 pyrimidine oxyde-3 dans 80 cm³ de méthanol chaud, on ajoute une solution de 4,23 g d'acide thiamorpholinone-3 oxyde-1 carboxylique-5 dans le minimum de méthanol bouillant.

On concentre le mélange réactionnel et on laisse refroidir. Le sel attendu cristallise sous forme de cristaux blancs dont le point de fusion est de 180°C (avec décomposition).

Analyse élémentaire : $C_{14}H_{22}N_6O_5S$

| | C% | H% | N% | O% | S% |
|---|---|---|---|---|---|
| Théorie : | 43,51 | 5,74 | 21,75 | 20,70 | 8,30 |
| Trouvé : | 43,42 | 5,77 | 21,96 | 20,84 | 8,27 |

EXEMPLE 6

Synthèse du sel de l'acide thiamorpholinone-3 dioxyde-1,1 carboxylique-5 et du pipéridino-6 diamino-2,4 pyrimidine oxyde-3

A une solution de 5 g de pipéridino-6 diamino-2,4 pyrimidine oxyde-3 dans 80 cm³ de méthanol chaud, on ajoute une solution de 4,61 g d'acide thiamorpholinone-3 dioxyde-1,1 carboxylique-5 dans le minimum de méthanol bouillant.

On concentre le mélange réactionnel à environ 75 cm³ et on laisse refroidir. Le produit est filtré, lavé au méthanol froid et séché. Le sel attendu cristallise sous forme de cristaux blancs dont le point de fusion est de 175°C.

Analyse élémentaire : $C_{14}H_{22}N_6O_6S$

| | C% | H% | N% | O% | S% |
|---|---|---|---|---|---|
| Théorie : | 41,78 | 5,51 | 20,88 | 23,85 | 7,97 |
| Trouvé : | 41,77 | 5,47 | 21,11 | 23,96 | 8,07 |

Exemples de formulations :

On prépare les trois lotions actives pour stimuler la croissance des cheveux de composition suivante :

EXEMPLE 1

| | | |
|---|---|---|
| - Sel de l'acide thiamorpholinone-3 carboxylique-5 et de pipéridino-6 diamino-2,4 pyrimidine oxyde-3 | | 0,85 g |
| - Hétéropolysaccharide vendu par la Société RHONE-POULENC sous la dénomination RHODOPOL 23 | | 1,00 g |
| - Conservateur qs | | |
| - Eau | qsp | 100,00 g |

## EXEMPLE 2

| | | |
|---|---|---|
| - Sel de l'acide méthyl-2 thiamorpholinone-3 carboxylique-5 et du pipéridino-6 diamino-2,4 pyrimidine oxyde-3 | | 5,00 g |
| - Acide polyacrylique réticulé de PM : 3.000.000, vendu sous la dénomination "CARBOPOL 934" par la Société GOODRICH | | 1,00 g |
| - Amino-2 méthyl-2 propanol-1 | qsp | pH 7,5 |
| - Eau | qsp | 100,00 g |

## EXEMPLE 3

| | | |
|---|---|---|
| - Sel de l'acide thiamorpholinone-3 oxyde-1 carboxylique-5 et du pipéridino diamino-2,4 pyrimidine oxyde-3 | | 2,50 g |
| - Hydroxypropylcellulose vendu sous la dénomination "KLUCEL G" par la Société HERCULES | | 3,00 g |
| - Conservateur | qs | |
| - Eau | qsp | 100,00 g |

1 à 2 ml de ces compositions, sous forme de lotions, sont appliqués sur la zone alopécique du cuir chevelu à une fréquence de deux applications par jour, en favorisant la pénétration par un massage.

**Revendications**

**Revendications pour les Etats Contractants : AT, BE, CH, DE, FR, GB, IT, LI, NL, SE**

1. Composé caractérisé par le fait qu'il répond à la formule :

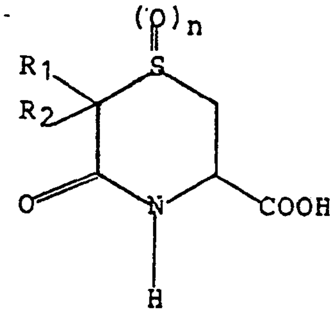

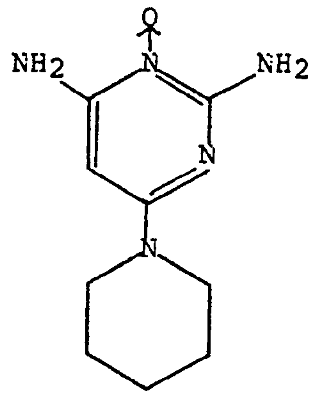

I(A)

ou à la forme ionique correspondante :

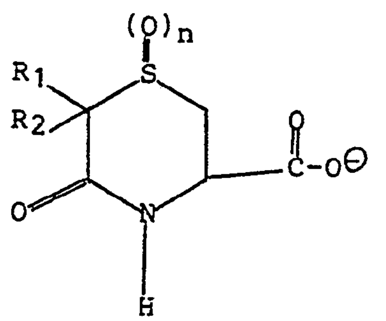

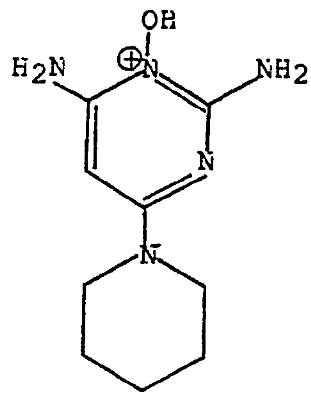

I(B)

dans laquelle :

n est égal à 0, 1 ou 2, et

$R_1$ et $R_2$ identiques ou différents, représentent un atome d'hydrogène ou un radical alkyle inférieur linéaire ou ramifié, ayant 1 à 6 atomes de carbone.

2. Composé selon la revendication 1, caractérisé par le fait que le radical alkyle inférieur représenté par $R_1$ et/ou $R_2$ est un radical méthyle, éthyle, propyle, isopropyle, butyle, pentyle, hexyle.

3. Composé selon la revendication 1 ou 2, caractérisé par le fait qu'il est choisi parmi le thiamorpholinone-3 carboxylate-5 de pipéridino-6 diamino-2,4 hydroxy-3 pyrimidinium, le méthyl-2 thiamorpholinone-3 carboxylate-5 de pipéridino-6 diamino-2,4 hydroxy-3 pyrimidinium, le diméthyl-2,2 thiamorpholinone-3 carboxylate-5 de pipéridino-6 diamino-2,4 hydroxy-3 pyrimidinium, l'oxo-1 thiamorpholinone-3 carboxylate-5 de pipéridino-6 diamino-2,4 hydroxy-3 pyrimidinium, le dioxo-1,1 thiamorpholinone-3 carboxylate-5 de pipéridino-6 diamino-2,4 hydroxy-3 pyrimidinium, l'oxo-1 méthyl-2 thiamorpholinone-3 carboxylate-5 de pipéridino-6 diamino-2,4 hydroxy-3 pyrimidinium, l'oxo-1 diméthyl-2,2 thiamorpholinone-3 carboxylate-5 pipéridino-6 diamino-2,4 hydroxy-3 pyrimidinium, l'hexyl-2 thiamorpholinone-3 carboxylate-5 de pipéridino-6 diamino-2,4 hydroxy-3 pyrimidinium.

4. Composition destinée à une application topique, caractérisée par le fait qu'elle contient dans un milieu aqueux approprié à l'application topique, 0,1 à 10% et de préférence 0,5 à 5% en poids par rapport au poids total de la composition d'un composé tel que défini dans l'une quelconque des revendications 1 à 3.

5. Composition selon la revendication 4, caractérisée par le fait qu'elle se présente sous forme de lotion, de crème, de gel ou qu'elle est conditionnée dans un dispositif aérosol.

6. Composition selon la revendication 4 ou 5, caractérisée par le fait qu'elle contient également du glycérol, de l'urée, de l'acide lactique ou des solvants.

7. Composition selon l'une quelconque des revendications 4 à 6, caractérisée par le fait qu'elle contient également la thiamorpholinone, la S-benzylcystéamine et ses dérivés, la tioxolone.

8. Procédé de traitement cosmétique du cuir chevelu, en vue d'induire et de stimuler la croissance des cheveux ou diminuer leur chute, caractérisé par le fait que l'on applique sur le cuir chevelu au moins un composé tel que défini dans l'une quelconque des revendications 1 à 3.

9. Composition pharmaceutique caractérisée par le fait qu'elle a la composition telle que définie dans l'une quelconque des revendications 4 à 7.

10. Utilisation des composés tels que définis dans l'une quelconque des revendications 1 à 3, pour la préparation d'une composition destinée au traitement de la chute des cheveux, de la pelade, des dermatites desquamantes, de l'alopécie et de la séborrhée.

**Revendications pour l'Etat Contractant : GR**

1. Composé caractérisé par le fait qu'il répond à la formule :

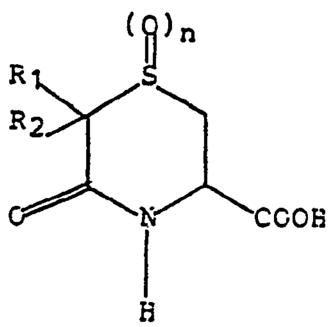

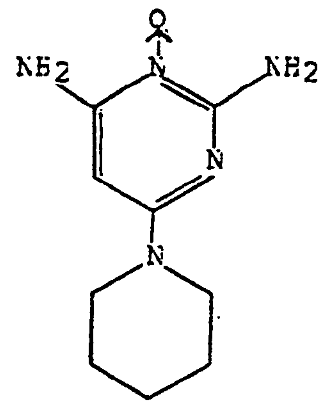

I(A)

ou à la formule ionique correspondante :

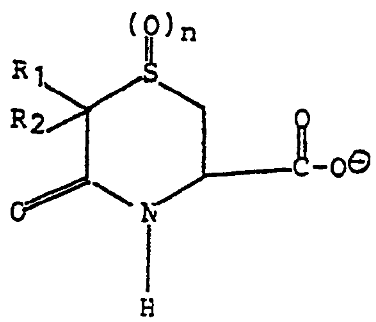

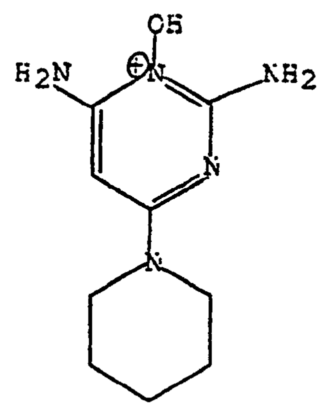

I(B)

dans laquelle :

n est égal à 0, 1 ou 2, et

$R_1$ et $R_2$ identiques ou différents, représentent un atome d'hydrogène ou un radical alkyle inférieur linéaire ou ramifié, ayant 1 à 6 atomes de carbone.

2. Composé selon la revendication 1, caractérisé par le fait que le radical alkyle inférieur représenté par $R_1$ et/ou $R_2$ est un radical méthyle, éthyle, propyle, isopropyle, butyle, pentyle, hexyle.

3. Composé selon la revendication 1 ou 2, caractérisé par le fait qu'il est choisi parmi le thiamorpholinone-3 carboxylate-5 de pipéridino-6 diamino-2,4 hydroxy-3 pyrimidinium, le méthyl-2 thiamorpholinone-3 carboxylate-5 de pipéridino-6 diamino-2,4 hydroxy-3 pyrimidinium, le diméthyl-2,2 thiamorpholinone-3 carboxylate-5 de pipéridino-6 diamino-2,4 hydroxy-3 pyrimidinium, l'oxo-1 thiamorpholinone-3 carboxylate-5 de pipéridino-6 diamino-2,4 hydroxy-3 pyrimidinium, le dioxo-1,1 thiamorpholinone-3 carboxylate-5 de pipéridino-6 diamino-2,4 hydroxy-3 pyrimidinium, l'oxo-1 méthyl-2 thiamorpholinone-3 carboxylate-5 de pipéridino-6 diamino-2,4 hydroxy-3 pyrimidinium, l'oxo-1 diméthyl-2,2 thiamorpholinone-3 carboxylite-5 pipéridino-6 diamino-2,4 hydroxy-3 pyrimidinium, l'hexyl-2 thiamorpholinone-3 carboxylate-5 de pipéridino-6 diamino-2,4 hydroxy-3 pyrimidinium.

4. Composition destinée à une application topique, caractérisée par le fait qu'elle contient dans un milieu aqueux approprié à l'application topique, 0,1 à 10% et de préférence 0,5 à 5% en poids par rapport au poids total de la composition d'un composé tel que défini dans l'une quelconque des revendications 1 à 3.

5. Composition selon la revendication 4, caractérisée par le fait qu'elle se présente sous forme de lotion, de crème, de gel ou qu'elle est conditionnée dans un dispositif aérosol.

6. Composition selon la revendication 4 ou 5, caractérisée par le fait qu'elle contient également du glycérol, de l'urée, de l'acide lactique ou des solvants.

7. Composition selon l'une quelconque des revendications 4 à 6, caractérisée par le fait qu'elle contient également la thiamorpholinone, la S-benzylcystéamine et ses dérivés, la tioxolone.

8. Procédé de traitement cosmétique du cuir chevelu, en vue d'induire et de stimuler la croissance des cheveux ou diminuer leur chute, caractérisé par le fait que l'on applique sur le cuir chevelu au moins un composé tel que défini dans l'une quelconque des revendications 1 à 3.

9. Utilisation des composés tels que définis dans l'une quelconque des revendications 1 à 3, pour la préparation d'une composition destinée au traitement de la chute des cheveux, de la pelade, des dermatites desquamantes, de l'alopécie et de la séborrhée.

**Revendications pour l'Etat Contractant : ES**

1. Procédé de préparation d'un composé répondant à la formule :

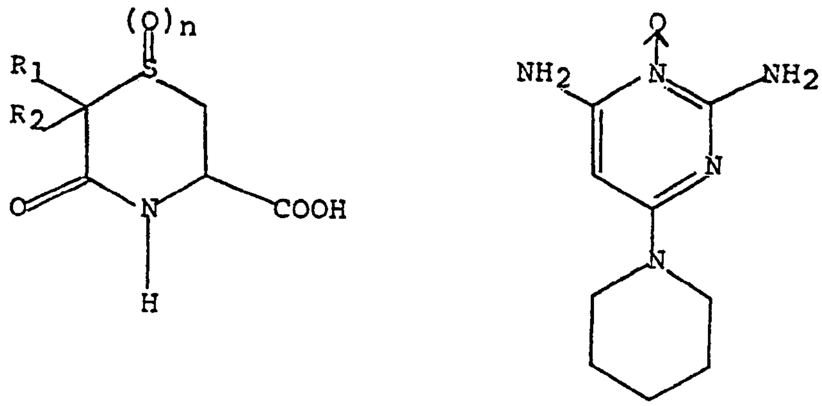

I(A)

ou à la forme ionique correspondante :

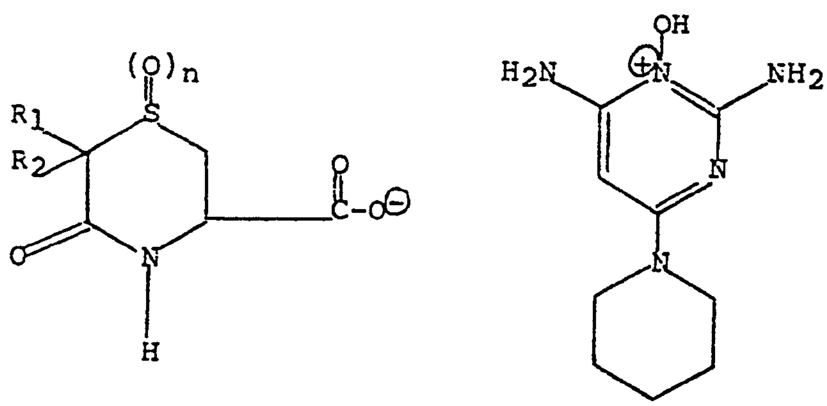

I(B)

dans laquelle :

n est égal à 0, 1 ou 2, et

$R_1$ et $R_2$, identiques ou différents, représentent un atome d'hydrogène ou un radical alkyle inférieur linéaire ou ramifié, ayant 1 à 6 atomes de carbone, caractérisé par le fait que l'on ajoute à une solution ou une suspension de pipéridino-6 diamino-2,4 pyrimidine oxyde-3 de l'acide thiamorpholinone carboxylique de formule :

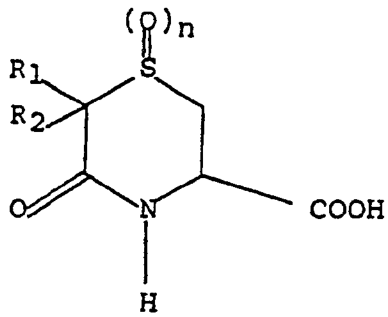

dans laquelle $R_1$ et $R_2$ ont les significations indiquées ci-dessus.

2. Procédé selon la revendication 1, caractérisé par le fait que l'acide thiamorpholino carboxylique est ajouté à une solution alcoolique de pipéridino-6 diamino-2,4 pyrimidine oxyde-3 sous forme de solution alcoolique.

3. Procédé selon la revendication 1 ou 2, caractérisé par le fait que le radical alkyle inférieur représenté par $R_1$ et/ou $R_2$ est un radical méthyle, éthyle, propyle, isopropyle, butyle, pentyle, hexyle.

4. Procédé selon l'une quelconque des revendications 1 à 3, caractérisé par le fait que l'acide est choisi parmi l'acide thiamorpholinone-3 carboxylique-5, l'acide méthyl-2 thiamorpholinone-3 carboxylique-5, l'acide diméthyl-2,2 thiamorpholinone-3 carboxylique-5, l'acide oxo-1 thiamorpholinone-3 carboxylique-5, l'acide dioxo-1,1 thiamorpholinone-3 carboxylique-5, l'acide oxo-1 méthyl-2 thiamorpholinone-3 carboxylique-5, l'acide oxo-1 diméthyl-2,2 thiamorpholinone-3 carboxylique-5, l'acide hexyl-2 thiamorpholinone-3 carboxylique-5.

5. Procédé de préparation d'une composition destinée à une application topique, caractérisé par le fait que l'on introduit dans un milieux aqueux approprié à l'application topique, 0,1 à 10% et de préférence 0,5 à 5% en poids par rapport au poids total de la composition d'un composé tel que préparé dans l'une quelconque des revendications 1 à 4.

6. Procédé selon la revendication 5, caractérisé par le fait que la composition se présente sous forme de lotion, de crème, de gel ou qu'elle est conditionnée dans un dispositif aérosol.

7. Procédé selon la revendication 5 ou 6, caractérisé par le fait que l'on introduit dans la composition également du glycérol, de l'urée, de l'acide lactique ou des solvants.

8. Procédé selon l'une quelconque des revendications 5 à 7, caractérisé par le fait que l'on introduit également dans la composition la thiamorpholinone, la S-benzylcystéamine et ses dérivés, la thioxolone.

9. Utilisation des composés préparés dans l'une quelconque des revendications 1 à 4, pour la préparation d'une composition destinée au traitement de la chute des cheveux, de la pelade, des dermatites desquamantes, de l'alopécie et de la séborrhée.

**Ansprüche**

**Patentansprüche für die Vertragsstaaten : AT, BE, CH, DE, FR, GB, IT, LI, NL, SE**

1. Verbindung, **gekennzeichnet** durch die Formel :

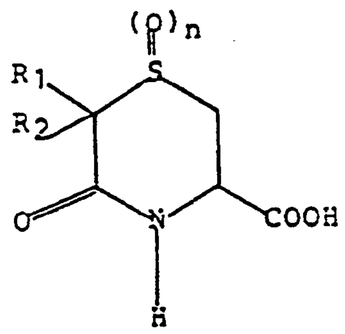 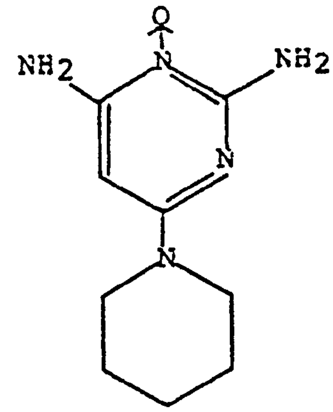 I(A)

oder die entsprechende ionische Form :

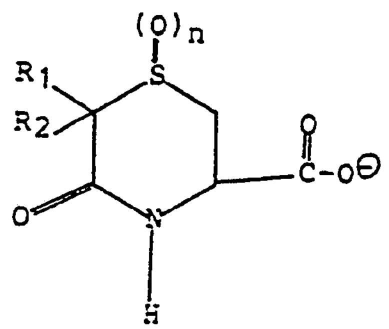 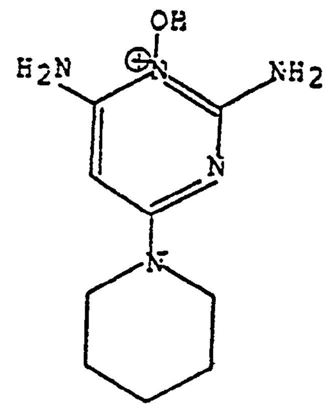 I(B)

worin n 0, 1 oder 2 ist und

$R_1$ und $R_2$, gleich oder verschieden, ein Wasserstoffatom oder einen linearen oder verzweigten Niedrigalkylrest mit 1 bis 6 Kohlenstoffatomen darstellen.

2. Verbindung gemäß Anspruch 1, dadurch **gekennzeichnet**, daß der durch $R_1$ und/oder $R_2$ dargestellte Niedrigalkylrest ein Methyl-, Ethyl-, Propyl-, Isopropyl-, Butyl-, Pentyl-, Hexylrest ist.

3. Verbindung gemäß Anspruch 1 oder 2, dadurch **gekennzeichnet**, daß sie unter Thiamorpholin-3-on-5-carboxylat, 2-Methylthiamorpholin-3-on-5-carboxylat, 2,2-Dimethylthiamorpholin-3-on-5-carboxylat, 1-Oxothiamorpholin-3-on-5-carboxylat, 1,1-Dioxothiamorpholin-3-on-5-carboxylat, 1-Oxo-2-methylthiamorpholin-3-on-5-carboxylat, 1-Oxo-2,2-dimethylthiamorpholin-3-on-5-carboxylat und 2-Hexylthiamorpholin- 3-on-5-carboxylat von 6-Piperidino-2,4-diamino-3-hydroxypyrimidinium ausgewählt ist.

4. Zusammensetzung zur örtlichen Aufbringung, dadurch **gekennzeichnet**, daß sie in einem zur örtlichen Aufbringung geeigneten wässrigen Medium 0,1 bis 10 Gewichtsprozent, vorzugsweise 0,5 bis 5 Gewichtsprozent, bezogen auf Gesamtgewicht der Zusammensetzung, einer Verbindung gemäß einem der Ansprüche 1 bis 3 enthält.

5. Zusammensetzung gemäß Anspruch 4, dadurch **gekennzeichnet**, daß sie in der Form einer Lotion, Creme, eines Gels vorliegt oder in einer Aerosol-Vorrichtung zubereitet ist.

6. Zusammensetzung gemäß Anspruch 4 oder 5, dadurch **gekennzeichnet**, daß sie auch Glycerin, Harnstoff, Milchsäure oder Lösungsmittel enthält.

7. Zusammensetzung gemäß jedem der Ansprüche 4 bis 6, dadurch **gekennzeichnet**, daß sie auch Thiamorpholinon, S-Benzylcysteamin und die Derivate davon, Thioxolon enthält.

8. Verfahren zur kosmetischen Behandlung von behaarter Haut, um das Wachstum von Haaren anzuregen und zu stimulieren oder deren Ausfall zu vermindern, dadurch **gekennzeichnet**, daß man auf die behaarte Haut mindestens eine Verbindung gemäß jedem der Ansprüche 1 bis 3 aufbringt.

9. Pharmazeutische Zusammensetzung, dadurch **gekennzeichnet**, daß sie die Zusammensetzung gemäß jedem der Ansprüche 4 bis 7 enthält.

10. Verwendung der Verbindungen gemäß jedem der Ansprüche 1 bis 3 zur Herstellung einer Zusammensetzung zur Behandlung von Haarausfall, Pelade, schuppenden Hautentzündungen, Alopezie und Seborrhoe.

**Patentansprüche für den Vertragsstaat : ES**

1. Verfahren zur Herstellung einer Verbindung der Formel :

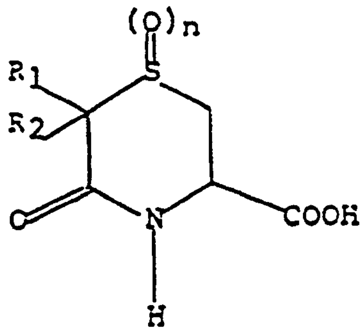

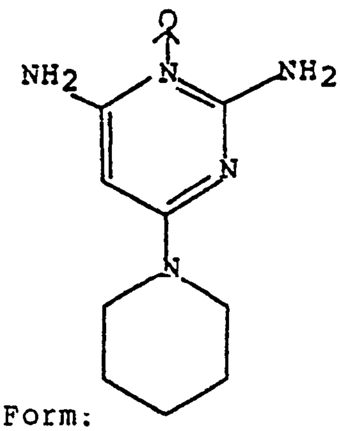

I(A)

oder der entsprechenden ionischen Form:

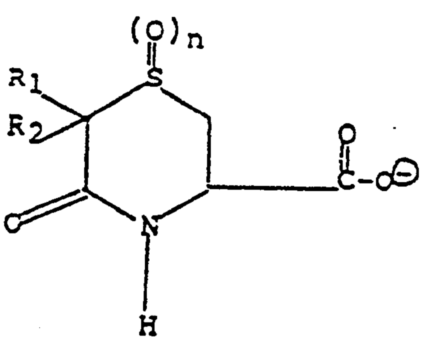

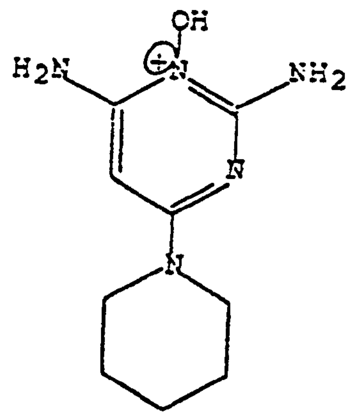

I(B)

worin n 0, 1 oder 2 ist und

$R_1$ und $R_2$, gleich oder verschieden, ein Wasserstoffatom oder einen linearen oder verzweigten Niedrigalkylrest mit 1 bis 6 Kohlenstoffatomen darstellen, dadurch **gekennzeichnet**, daß man zu einer Lösung oder Suspension von 6-Piperidino-2,4-diaminopyrimidin-3-oxid Thiamorpholinoncarbonsäure der Formel gibt :

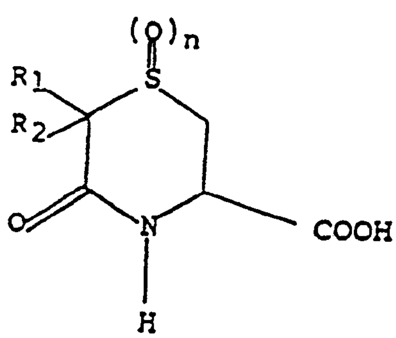

worin $R_1$ und $R_2$ die oben angegebenen Bedeutungen haben.

2. Verfahren gemäß Anspruch 1, dadurch **gekennzeichnet**, daß man die Thiamorpholinoncarbonsäure zu einer alkoholischen Lösung von 6-Piperidino-2,4-diaminopyrimidin-3-oxid in der Form einer alkoholischen Lösung gibt.

3. Verfahren gemäß Anspruch 1 oder 2, dadurch **gekennzeichnet**, daß der durch $R_1$ und/oder $R_2$ dargestellte Niedrigalkylrest ein Methyl-, Ethyl-, Propyl-, Isopropyl-, Butyl-, Pentyl-, Hexylrest ist.

4. Verfahren gemäß jedem der Ansprüche 1 bis 3, dadurch **gekennzeichnet**, daß die Säure unter Thiamorpholin-3-on-5-carbonsäure, 2-Methylthiamorpholin-3-on-5-carbonsäure, 2,2-Dimethylthiamorpholin-3-on-5-carbonsäure, 1-Oxothiamorpholin-3-on-5-carbonsäure, 1,1-Dioxothiamorpholin-3-on-5-carbonsäure,

1-Oxo-2-methylthiamorpholin-3-on-5-carbonsäure, 1-Oxo-2,2-dimethylthiamorpholin-3-on-5-carbonsäure und 2-Hexylthiamorpholin-3-on-5-carbonsäure ausgewählt ist.

5. Verfahren zur Herstellung einer Zusammensetzung zur örtlichen Aufbringung, dadurch **gekennzeichnet**, daß man in ein zur örtlichen Aufbringung geeignetes wässriges Medium 0,1 bis 10 Gewichtsprozent, vorzugsweise 0,5 bis 5 Gewichtsprozent, bezogen auf Gesamtgewicht der Zusammensetzung, einer Verbindung gemäß jedem der Ansprüche 1 bis 4 gibt.

6. Verfahren gemäß Anspruch 5, dadurch **gekennzeichnet**, daß die Zusammensetzung in der Form einer Lotion, Creme, eines Gels vorliegt oder in einer Aerosol-Vorrichtung zubereitet ist.

7. Verfahren gemäß Anspruch 5 oder 6, dadurch **gekennzeichnet**, daß man der Zusammensetzung auch Glycerin, Harnstoff, Milchsäure oder Lösungsmittel zufügt.

8. Verfahren gemäß jedem der Ansprüche 5 bis 7, dadurch **gekennzeichnet**, daß man der Zusammensetzung auch Thiamorpholinon, S-Benzylcysteamin und die Derivate davon, Thioxolon zufügt.

9. Verwendung der gemäß jedem der Ansprüche 1 bis 4 hergestellten Verbindungen zur Herstellung einer Zusammensetzung zur Behandlung von Haarausfall, Pelade, schuppenden Hautentzündungen, Alopezie und Seborrhoe.

**Patentansprüche für den Vertragsstaat : GR**

1. Verbindung, **gekennzeichnet** durch die Formel :

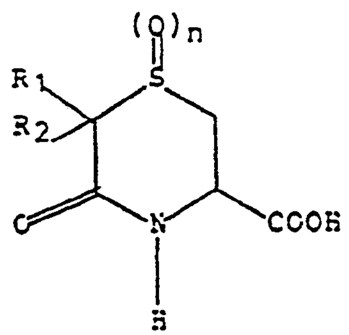

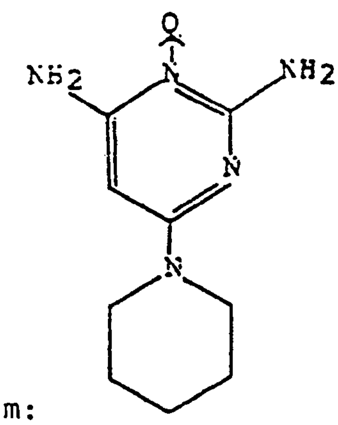

I(A)

oder die entsprechende ionische Form:

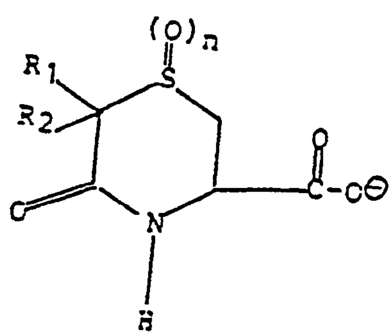

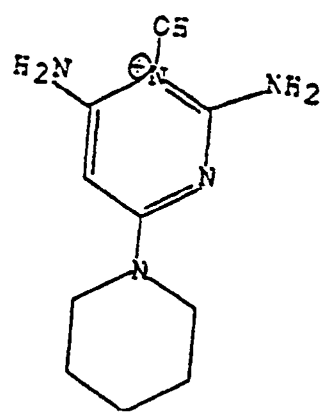

I(B)

worin n 0, 1 oder 2 ist und

$R_1$ und $R_2$, gleich oder verschieden, ein Wasserstoffatom oder einen linearen oder verzweigten Niedrigalkylrest mit 1 bis 6 Kohlenstoffatomen darstellen.

2. Verbindung gemäß Anspruch 1, dadurch **gekennzeichnet**, daß der durch $R_1$ und/oder $R_2$ dargestellte Niedrigalkylrest ein Methyl-, Ethyl-, Propyl-, Isopropyl-, Butyl-, Pentyl-, Hexylrest ist.

3. Verbindung gemäß Anspruch 1 oder 2, dadurch **gekennzeichnet**, daß sie unter Thiamorpholin-3-on-5-carboxylat, 2-Methylthiamorpholin-3-on-5-carboxylat, 2,2-Dimethylthiamorpholin-3-on-5-carboxylat, 1-Oxothiamorpholin-3-on-5-carboxylat, 1,1-Dioxothiamorpholin-3-on-5-carboxylat, 1-Oxo-2-methylthiamorpholin-3-on-5-carboxylat, 1-Oxo-2,2-dimethylthiamorpholin-3-on-5-carboxylat und 2-Hexylthiamorpholin- 3-on-5-carboxylat von 6-Piperidino-2,4-diamino-3-hydroxypyrimidinium ausgewählt ist.

4. Zusammensetzung zur örtlichen Aufbringung, dadurch **gekennzeichnet**, daß sie in einem zur örtlichen Aufbringung geeigneten wässrigen Medium 0,1 bis 10 Gewichtsprozent, vorzugsweise 0,5 bis 5 Gewichtsprozent, bezogen auf Gesamtgewicht der Zusammensetzung, einer Verbindung gemäß einem der Ansprüche 1 bis 3 enthält.

5. Zusammensetzung gemäß Anspruch 4, dadurch **gekennzeichnet**, daß sie in der Form einer Lotion, Creme, eines Gels vorliegt oder in einer Aerosol-Vorrichtung zubereitet ist.

6. Zusammensetzung gemäß Anspruch 4 oder 5, dadurch **gekennzeichnet**, daß sie auch Glycerin, Harnstoff, Milchsäure oder Lösungsmittel enthält.

7. Zusammensetzung gemäß jedem der Ansprüche 4 bis 6, dadurch **gekennzeichnet**, daß sie auch Thiamorpholinon, S-Benzylcysteamin und die Derivate davon, Thioxolon enthält.

8. Verfahren zur kosmetischen Behandlung von behaarter Haut, um das Wachstum von Haaren anzuregen und zu stimulieren oder deren Ausfall zu vermindern, dadurch **gekennzeichnet**, daß man auf die behaarte Haut mindestens eine Verbindung gemäß jedem der Ansprüche 1 bis 3 aufbringt.

9. Verwendung der Verbindungen gemäß jedem der Ansprüche 1 bis 3, zür Herstellung einer Zusammensetzung zur Behandlung von Haarausfall, Pelade, schuppenden Hautentzündungen, Alopezie und Seborrhoe.

## Claims

### Claims for the Contracting States : AT, BE, CH, DE, FR, GB, IT, LI, NL, SE

1. Compound, characterized in that it corresponds to the formula :

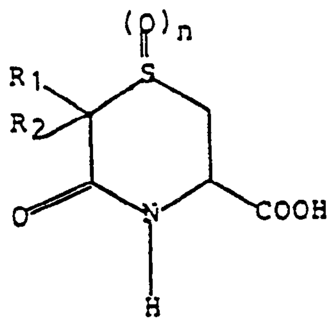
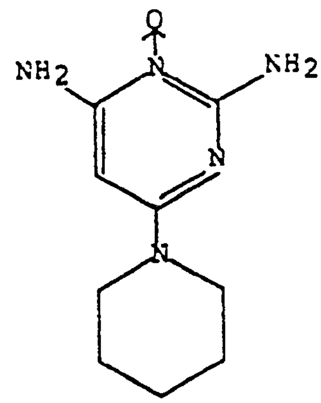

I(A)

or to the corresponding ionic form :

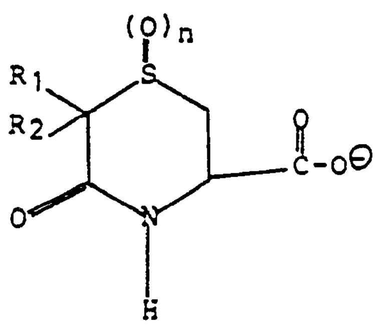
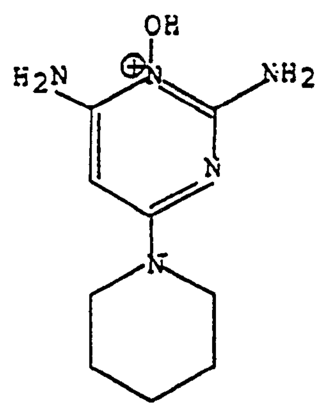

I(B)

in which :

n is equal to 0, 1 or 2, and

$R_1$ and $R_2$, which may be identical or different, represent a hydrogen atom or a linear or branched lower alkyl radical having 1 to 6 carbon atoms.

2. Compound according to Claim 1, characterized in that the lower alkyl radical represented by $R_1$ and/or $R_2$ is a methyl, ethyl, propyl, isopropyl, butyl, pentyl or hexyl radical.

3. Compound according to Claim 1 or 2, characterized in that it is selected from 6-piperidino-2,4-diamino-3-hydroxypyrimidinium 3-thiamorpholinone-5-carboxylate, 6-piperidino-2,4-diamino-3-hydroxypyrimidinium 2-methyl-3-thiamorpholinone-5-carboxylate, 6-piperidino-2,4-diamino-3-hydroxypyrimidinium 2,2-dimethyl-3-thiamorpholinone-5-carboxylate, 6-piperidino-2,4-diamino-3-hydroxypyrimidinium 1-oxo-3-thiamorpholinone- 5-carboxylate, 6-piperidino-2,4-diamino-3-hydroxypyrimidinium 1,1-dioxo-3-thiamorpholinone-5-carboxylate, 6-piperidino-2,4-diamino-3-hydroxypyrimidinium 1-oxo-2-methyl-3-thiamorpholinone-5-carboxylate, 6-piperidino-2,4-diamino-3-hydroxypyrimidinium 1-oxo-2,2-dimethyl-3-thiamorpholinone-5-carboxylate and 6-piperidino-2,4-diamino-3-hydroxypyrimidinium 2-hexyl-3-thiamorpholinone-5-carboxylate.

4. Composition intended for a topical application, characterized in that it contains, in an aqueous medium suitable for topical application, 0.1 to 10%, and preferably 0.5 to 5%, by weight relative to the total weight of the composition, of a compound as defined in any one of Claims 1 to 3.

5. Composition according to Claim 4, characterized in that it is presented in the form of a lotion, cream or gel, or in that it is packaged in an aerosol device.

6. Composition according to Claim 4 or 5, characterized in that it also contains glycerol, urea, lactic acid or solvents.

7. Composition according to any one of Claims 4 to 6, characterized in that it also contains thiamorpholinone, S-benzylcysteamine and its derivatives or tioxolone.

8. Process for cosmetic treatment of the scalp for the purpose of inducing and stimulating hair growth or decreasing its loss, characterized in that at least one compound as defined in any one of Claims 1 to 3 is applied on the scalp.

9. Pharmaceutical composition, characterized in that it has the composition as defined in any one of Claims 4 to 7.

10. Use of the compounds as defined in any one of Claims 1 to 7, for the preparation of a composition intended for the treatment of hair loss, pelade, desquamating dermatitis, alopecia and seborrhoea.

**Claims for the Contracting State : ES**

1. Process for preparing a compound corresponding to the formula :

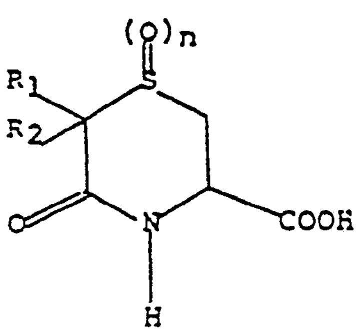

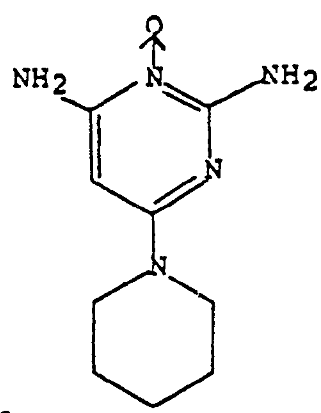

I(A)

or to the corresponding ionic form:

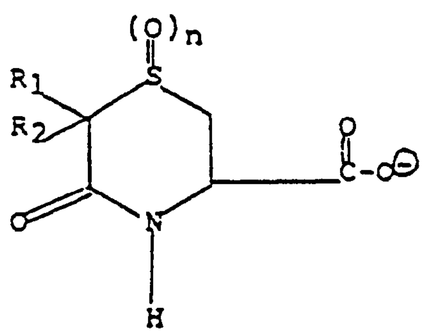

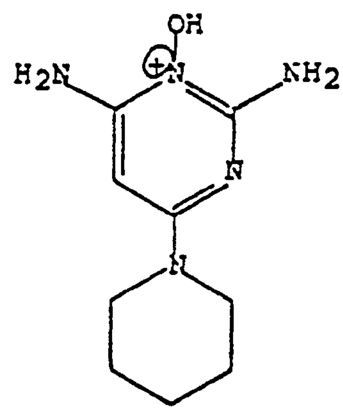

I(B)

in which :

n is equal to 0, 1 or 2, and

$R_1$ and $R_2$, which may be identical or different, represent a hydrogen atom or a linear or branched lower alkyl radical having 1 to 6 carbon atoms, characterized in that a thiamorpholinonecarboxylic acid of formula:

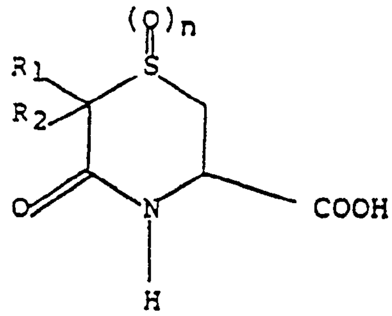

in which $R_1$ and $R_2$ have the meanings stated above, is added to a solution or a suspension of 6-piperidino-2,4-diaminopyrimidine 3-oxide.

2. Process according to Claim 1, characterized in that the thiamorpholinonecarboxylic acid is added to an alcoholic solution of 6-piperidino-2,4-diaminopyrimidine 3-oxide in the form of an alcoholic solution.

3. Process according to Claim 1 or 2, characterized in that the lower alkyl radical represented by $R_1$ and/or $R_2$ is a methyl, ethyl, propyl, isopropyl, butyl, pentyl or hexyl radical.

4. Process according to any one of Claims 1 to 3, characterized in that the acid is selected from 3-thiamorpholinone-5-carboxylic acid, 2-methyl-3-thiamorpholinone-5-carboxylic acid, 2,2-dimethyl-3-thiamorpholinone-5-carboxylic acid, 1-oxo-3-thiamorpholinone-5-carboxylic acid, 1,1-dioxo-3-thiamorpholinone-5-carboxylic acid, 1-oxo-2-methyl-3-thiamorpholinone-5-carboxylic acid, 1-oxo-2,2-dimethyl-3-thiamorpholinone-5-carboxylic acid and 2-hexyl-3-thiamorpholinone-5-carboxylic acid.

5. Process for preparing a composition intended for a topical application, characterized in that 0.1 to 10%, and preferably 0.5 to 5%, by weight relative to the total weight of the composition, of a compound as prepared in any one of Claims 1 to 4 is introduced into an aqueous medium suitable for topical application.

6. Process according to Claim 5, characterized in that the composition is presented in the form of a lotion, cream or gel, or in that it is packaged in an aerosol device.

7. Process according to Claim 5 or 6, characterized in that glycerol, urea, lactic acid or solvents is/are also introduced into the composition.

8. Process according to any one of Claims 5 to 7, characterized in that thiamorpholinone, S-benzylcysteamine and its derivatives or tioxolone is also introduced into the composition.

9. Use of the compounds prepared in any one of Claims 1 to 4, for the preparation of a composition intended for the treatment of hair loss, pelade, desquamating dermatitis, alopecia and seborrhoea.

**Claims for the Contracting State : GR**

1. Compound, characterized in that it corresponds to the formula :

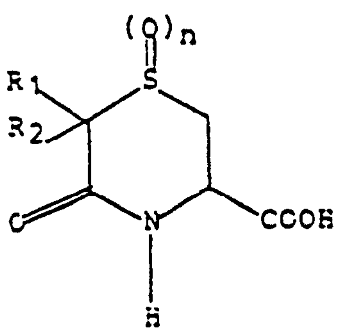

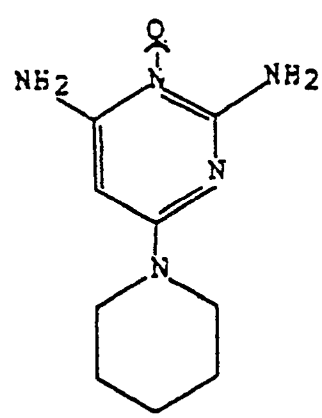

I(A)

or to the corresponding ionic form :

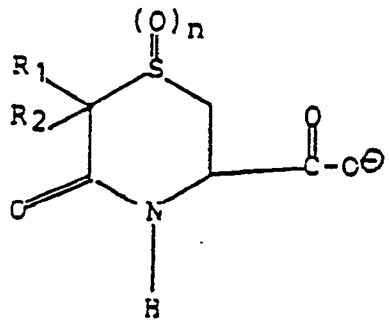

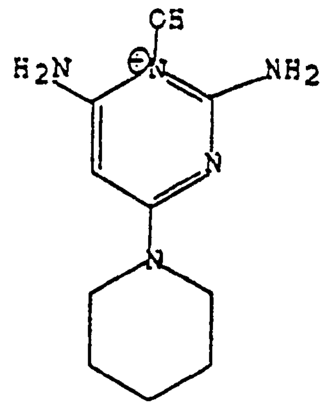

I(B)

in which :

n is equal to 0, 1 or 2, and

$R_1$ and $R_2$, which may be identical or different, represent a hydrogen atom or a linear or branched lower alkyl radical having 1 to 6 carbon atoms.

2. Compound according to Claim 1, characterized in that the lower alkyl radical represented by $R_1$ and/or $R_2$ is a methyl, ethyl, propyl, isopropyl, butyl, pentyl or hexyl radical.

3. Compound according to Claim 1 or 2, characterized in that it is selected from 6-piperidino-2,4-diamino-3-hydroxypyrimidinium 3-thiamorpholinone-5-carboxylate, 6-piperidino-2,4-diamino-3-hydroxyrimidinium 2-methyl-3-thiamorpholinone-5-carboxylate, 6-piperidino-2,4-diamino-3-hydroxypyrimidinium 2,2-dimethyl-3-thiamorpholinone-5-carboxylate, 6-piperidino-2,4-diamino-3-hydroxypyrimidinium 1-oxo-3-thiamorpholinone-5-carboxylate,6-piperidino-2,4-diamino-3-hydroxypyrimidinium 1,1-dioxo-3-thiamorpholinone-5-carboxylate, 6-piperidino-2,4-diamino-3-hydroxypyrimidinium 1-oxo-2-methyl-3-thiamorpholinone-5-carboxylate, 6-piperidino-2,4-diamino-3-hydroxypyrimidinium 1-oxo-2,2-dimethyl-3-thiamorpholinone-5-carboxylate and 6-piperidino-2,4-diamino-3-hydroxypyrimidinium 2-hexyl-3-thiamorpholinone-5-carboxylate.

4. Composition intended for a topical application, characterized in that it contains, in an aqueous medium suitable for topical application, 0.1 to 10%, and preferably 0.5 to 5%, by weight relative to the total weight of the composition, of a compound as defined in any one of Claims 1 to 3.

5. Composition according to Claim 4, characterized in that it is presented in the form of a lotion, cream or gel, or in that it is packaged in an aerosol device.

6. Composition according to Claim 4 or 5, characterized in that it also contains glycerol, urea, lactic acid or solvents.

7. Composition according to any one of Claims 4 to 6, characterized in that it also contains thiamorpholinone, S-benzylcysteamine and its derivatives or tioxolone.

8. Process for cosmetic treatment of the scalp for the purpose of inducing and stimulating hair growth or decreasing its loss, characterized in that at least one compound as defined in any one of Claims 1 to 3 is applied on the scalp.

9. Use of the compounds as defined in any one of Claims 1 to 3, for the preparation of a composition intended for the treatment of hair loss, pelade, desquamating dermatitis, alopecia and seborrhoea.